# EUROPEAN PATENT APPLICATION

(11) **EP 4 082 502 A1**
(43) Date of publication of application: **02.11.2022**
(21) Application number: 22170396.0
(22) Date of filing: 27.04.2022
(51) Int. Cl.: A61G 7/10, A61G 13/12, G16H 20/00, A61G 7/057

(54) **METHOD AND SYSTEM OF MATTRESSES TO PUT THE PATIENT IN PRONE POSITION**

(30) Priority: 28.04.2021 PL 43773521
(71) Applicant: Uniwersytet Medyczny w Lublinie, 20-059 Lublin (PL)
(72) Inventor: DABROWSKI, Wojciech, 20-050 Lublin (PL)

(57) **Abstract**

Present invention relates to the method and system of mattresses for patient prone positioning. The method consists in placing next to the patient's body - anteriorily, a main mattress (1) by the head, a chest mattress (2) by the chest, the pelvic mattress (3) by the pelvis, the shin mattress (4) by the shins, and at least some of the mattresses used (1, 2, 3, 4) have a separate bed-adjoining cell (7) and a separate body-adjoining cell (8); then a body-adjoining cells (8) are inflated to the state of matching their shape to the patient's body and the patient is placed in the initial prone position, and then the bed-adjoining cells (7) are inflated, evenly lifting the individual parts of the body until the influence of the abdominal compression pressure is eliminated. Optionally, the system is equipped with a system measuring pressure in artificial airways (12), and independently of that, a pressure pulse generator (18).

## Description

The present invention relates to a method and a mattress system for prone positioning of patients, in particular for treating patients with severe respiratory failure, acute respiratory distress syndrome - ARDS, and also COVID-19.

Treatment of severe respiratory failure and ARDS requires advanced ventilation techniques, which do not always produce the desired results in a patient in supine position. Significant improvement can be achieved by prone positioning of the patient. In 2012, the World Society for Intensive Care issued recommendations in which prone position - the position on abdomen - took a key role in the treatment of severe respiratory failure. Since then, artificial lung ventilation in the prone position has become the gold standard of care for patients with severe respiratory failure and ARDS. This position has also become a key element in the treatment of COVID-19 patients. It turned out that the correct positioning of the patient in the discussed position allows not only for improving the quality of gas exchange, but also significantly reduces the need to implement advanced ventilation techniques and the extracorporeal membrane oxygenation method (ECMO). Therefore, this position is widely used in all departments treating patients with COVID-19 and patients with acute respiratory distress syndrome (ARDS).

Sponge pads are used with patient prone positioning in everyday clinical practice, the pads most often being significantly and unfavorably squashed under the influence of body weight. Furthermore, from the patent application DE 102009004604 A1, a mattress system is known for patient prone positioning. By means of the above-mentioned system of mattresses, a method of prone positioning is implemented, consisting in placing four inflatable mattresses next to the patient's body - anteriorly, i.e. the main mattress by the head, the chest mattress by the chest, pelvic mattress by the pelvis, shin mattress by the shins. At the same time, it is possible to adjust the mattress inflation to the patient's parameters.

Prone positioning of the conscious patient is not easy, especially in cases of coexisting obesity. Improper patient positioning and excessive abdominal pressure may be counterproductive. Too much compression of abdominal compliance results in decreased compliance of the lungs, and thus in deterioration of ventilation conditions. These changes are particularly important in patients requiring artificial lung ventilation, whose proper positioning must take into account the arching of the abdominal cavity above the chest level. At present, there are no devices that would eliminate abdominal compression pressure, to safely place the patient in the prone position.

The technical problem to be solved is prone positioning of the patient and at the same time reducing the abdominal compression pressure. This problem also includes not inducing a abdominal compression pressure effect during the prone positioning process itself. In particular, the problem concerns prone positioning of the conscious patient with coexisting obesity. Another technical problem is the additional stimulation of the chest in the prone position - in order to eliminate the residual secretions from the small bronchioles, which reduces the need for frequent patting of the patient's lungs.

The above technical problem is solved by the method and device according to the invention. The method of prone positioning of the patient according to the invention consists in placing the mattress next to the patient's body - anteriorly, a main mattress by the head, a chest mattress by the chest, a pelvic mattress by the pelvis, a shin mattress by the shins, the method according to the invention is characterized in that at least some of the mattresses used have a separate outer air cell and a separate body-adjoining air cell. Then, the body-adjoining cells are inflated to the point of matching their shape to the patient's body and the patient is placed in the initial prone position, and then the basal air cells are inflated, evenly lifting individual parts of the body until the abdominal compression pressure is eliminated.

A preferred variant of this method is when, before prone positioning of the patient and during inflating the outer cells, the intra-abdominal pressure is measured with the pressure measuring system in artificial airways, and the outer cells are inflated to an acceptably small difference between these intra-abdominal pressure values - due to medical conditions.

Another advantageous variant is that an aero pulse-alternating pressure is applied to the chest air cell on a chest mattress having a body-adjoining cell, which contributes to the elimination of residual secretions from the fine bronchioles, which in turn reduces the need for frequent patting of the patient's lungs. In particular, the aero pulse alternating pressure is applied every minute for a period of 10 to 15 seconds.

It is particularly advantageous if, after positioning the mattresses against the patient's body, they are fastened by enveloping the patient's body parts with fastening straps or when the mattresses are attached to a positioning ladder which is then attached to the patient's body.

A completely different variant of the method according to the invention is the method distinguished by the fact that inflation of the outer cells and / or the body-adjoining cells in mattress systems in which the pressure controller is connected to a central unit, which is a computer, is achieved until the pressure value read from the database is reached , in which there are values of the pressures of the body-adjoining and outer cells for people previously prone positioned with the use of mattresses. The read pressure values apply to people with the most similar values of weight and height within the same sex in relation to the set values of weight and height of the patient currently prone positioned.

An additional variant of the method according to the invention is distinguished by the fact that after inflating of the body-adjoining cells and outer cells, the pressure in these cells is adjusted on the basis of observation of the patient's condition.

Accordingly, the subject of the invention is also a mattress system for prone positioning of the patient, consisting of the main, chest, pelvic and shin inflatable mattresses - characterized in that these mattresses are connected with a pressure controller attached to the source pressure. Thereby, at least some of these mattresses have a separate outer cell and a separate body-adjoining cell, and each of the body-adjoining and outer cells is separately connected to a pressure controller.

In one of the variants, the main, chest and pelvic mattresses have a separate outer cell and a separate body-adjoining cell. The body-adjoining cell of the chest mattress consists of: the pre-jugular cell, the chest cell, and the pre-abdominal cell.

In a preferred embodiment, the pressure controller consists of a controller connected to the pressure sensor assembly as well as the valve assembly and the pressure source, and the pressure source is connected to the pressure sensor assembly.

In another variant, a pressure pulse generator is connected to the cell, and in a particularly preferred embodiment, the pressure pulse generator is contained in a pressure controller.

A very advantageous embodiment is when the artificial airway pressure measuring system is connected to the pressure controller, in particular when the artificial airway pressure measuring system is a peak airway pressure sensor.

In another preferred variant, the main mattress is of a horseshoe shape, the arms of which are connected by a spacer strip - which enables the adjustment of the spacing of these arms. In yet another variation, the pelvic mattress and the shin mattress are in a cylinder shape - including an elliptical cylinder.

It is also advantageous for the mattresses to have fastening straps, but also for the mattresses to be adjustable to a positioning ladder having the said fastening straps.

It is preferred that the mattresses are connected to the pressure controller through conduits divided according to the number of connected body-adjoining and outer cells - including the pre-jugular, chest and pre-abdominal cells.

It is also advantageous if the spacer strip and the fastening tapes have Velcro.

A particularly advantageous variant is characterized by the fact that each mattress is connected and integrated with a separate pressure controller which is connected and integrated with a separate pressure source, each pressure controller having its own autonomous source of electric energy, and each pressure controller having a transceiver module, through which it is wirelessly connected to the central unit.

In another embodiment, the pressure source is a compressor and a cartridge containing pressurized carbon dioxide.

In yet another but preferred embodiment, the central unit is a smartphone.

The advantages of the method and system according to the invention are that they ensure safe and, in particular, even lifting of the individual parts of the body in such a way that it does not induce abdominal compression pressure. This safety is also influenced by the fact that the mattresses are attached to the patient's body, which prevents the patient from slipping. The present invention allows for a completely safe prone positioning of the patient with adequate relief of abdominal compression pressure, which is crucial in this type of position, in particular in the treatment of ARDS and COVID-19. The division of individual mattresses into two cells is so advantageous that it is possible to inflate them with a different pressure, which translates into better performance of their various functions. The outer cells lift individual parts of the patient - which is associated with a higher pressure value, and the body-adjoining cells stabilize the patient's position - which does not require such a high pressure value. In addition, the chest cell of the chest mattress is supplied with pulse alternating pressure, which significantly improves elimination of residual secretions from fine bronchioles and reduces the need for frequent patting of the patient's lungs. An additional advantage is the compact version of the system according to the invention, because it is completely wireless, both in terms of electric and pneumatic cables, which facilitates work as there is no possibility of cables being tangled of this device.

The method and system according to the invention are shown in the drawing, in which: Fig. 1 shows the process of lifting a patient using the method according to the invention with still unsuppressed abdominal compression pressure; Fig. 2 shows the process of lifting a patient using the method according to the invention with the suppressed abdominal compression pressure; Fig. 3 shows an exemplary cross-section of a duct for a four-cell mattress - under magnification; Fig.4 shows a diagrammatic lateral view of the system according to the invention; Fig.5 shows a schematic view of the system according to the invention in top view; Fig.6 shows a block diagram of a pressure controller; Fig. 7 shows a fixing ladder; Fig.8 shows a block diagram of a compact version of the system according to the invention.

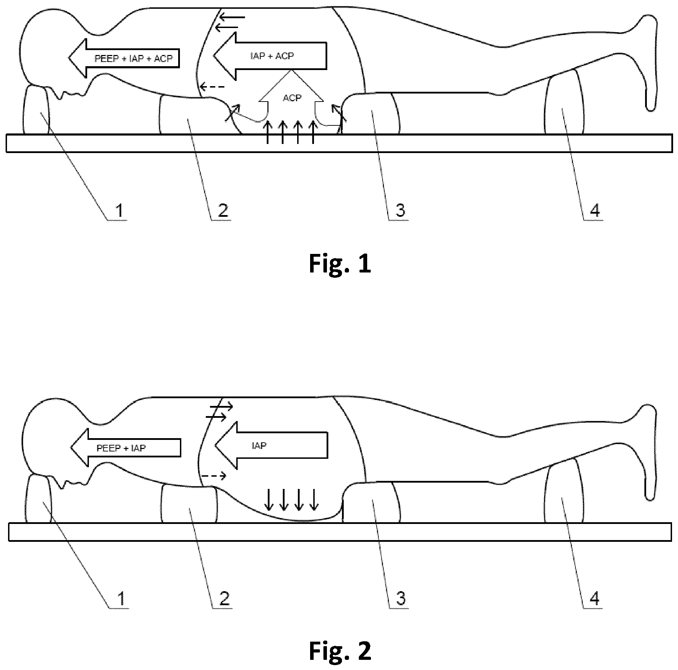

The method of patient prone positioning in the first embodiment consists in placing anteriorly mattresses 1, 2, 3, 4, next to the patient's body in a horizontal position , the shape of which is dedicated to individual parts of the body . As shown in Fig. 1: the main mattress 1 is placed by the head of the patient, the chest mattress 2 - by the chest, the pelvic mattress 3 - by the pelvis, the shin mattress 4 -by the shins. Mattresses 1, 2, 3, 4 are connected to a pressure controller 5 connected to a pressure source 6. The mattresses 1, 2, 3 have a separate outer cell 7, which after placing any of the mattresses 1, 2, 3 next to the patient's body is at the top - when the patient is still in a horizontal position, and when turned to the prone position, the outer cell 7 is at the bottom being in contact with the bed. Moreover, the mattresses 1, 2, 3 have a separate body-adjoining cell 8, which is located at the bottom - when the patient is still in a horizontal position, and when turned to the prone position, it is at the top. The mattresses 1, 2, 3 are attached to the patient's body by means of fastening straps 9 by strapping the appropriate body parts - accordingly to the mattresses 1, 2, 3. Optionally, the shin mattress 4 is also equipped with fastening straps 9. In particular, when fastening of the patient's body with fastening straps 9 in the places corresponding to the target positions of the mattresses 1, 2, 3 would be difficult or impossible (e.g. due to an existing injury or a surgery performed or planned at this place), an alternative method of fixing mattresses 1, 2, 3, 4 is used. It consists in that the mattresses 1, 2, 3, 4 are first attached to the positioning ladder 10, which is then attached to the patient's body by means of fastening straps 9 (which the positioning ladder 10 is also equipped with),the body parts, not necessarily corresponding to the exact positions of the mattresses 1, 2, 3, 4. The main mattress 1 is of a horseshoe shape, the arms of which are connected by a spacer strip 11 and the spacing of these arms is adjusted to fit the patient's head. Then, when the patient is in a horizontal position, the body-adjoining cells 8 are inflated to match their shape to the patient's body shape, which ensures patient's stable position after being turned to the prone position. Thereafter, the patient is initially rotated to the prone position and the outer surface cells 7 are inflated, evenly lifting the individual body parts until - as shown in Fig. 2, getting the effect of suppressing the abdominal compression pressure. Naturally, the value of the pressure in a given outer surface cell 7 is higher than the value of the body-adjoining cell 8, separate from it, and in the same mattress 1, 2, 3. The shin cell 4, on the other hand, has one cell (7, 8), which is both a outer-surface cell 7 and a body-adjoining cell 8.

Moreover, in Fig. 1 and Fig. 2, the sum of the pressures is symbolically represented, i.e. PEEP + IAP + ACP in Fig. 1 and PEEP + IAP in Fig. 2, where: PEEP (positive end-expiratory pressure) means positive pressure in the airways during the final expiration phase; IAP (intra-abdominal presure) is the mean pressure in the abdominal cavity; and ACP (abdominal compression pressure) is the pressure exerted by the bed surface on the abdominal cavity.

The second embodiment of the method according to the invention differs from the first in that before the patient is initially placed in the prone position and during the inflation of the outer-cells 7, the airway expiratory pressure is measured by connecting a pressure measuring system to the artificial airway 12.The outer cells 7 are inflated to an acceptably small - due to medical conditions - difference between the values measured in the horizontal position and the prone position.

The third embodiment of the method according to the invention proceeds in the same way as the previous examples, with the difference, however, that the chest mattress 2 used has a body-adjacent cell 8, which has a pre-jugular cell 13, a chest cell 14 and a pre-abdominal cell 15, and a pulse alternating pressure is applied to the chest cell 14, every minute for 10 or 15 seconds - causing the air to vibrate.

The fourth embodiment of the method according to the invention is distinguished from the other examples in that, notwithstanding the fact that the entire patient prone positioning process is supervised by personnel, the inflation of cells 7, 8 is automated by using the data of the previously positioned persons according to the invention. To implement the method according to the present example, a mattress system 1, 2, 3, 4 is needed in which the pressure controller 5 is connected to a central unit 16, which is a computer, in a particular case a smartphone. The inflation of the outer cells 7 and the body-adjoining cells 8 (although there is a variant of this example in which the automation relates to the inflation of only one of the cells 7, 8) is carried out in such a way that any of the cells 7, 8 is inflateded to the pressure in this cell 7, 8 equal to the pressure value obtained from the database for people with the closest weight and height within the same sex. The pressure value obtained from the database may be the value used in the previous prone positioning of a given patient, as well as it may be close to the values used in people with the most similar parameters, e.g. the average of the pressures of 5 people with the most similar parameters or the average of the pressures of people whose each parameter is at least 0.9 values of the parameters of the patient prone positioned according to the invention.

In cases where, e.g. due to the patient's individual characteristics, the pressure value in individual cells 7, 8 requires correction, the personnel supervising the patient's prone positioning makes an appropriate correction by inflating or partial deflating of the cells 7, 8.

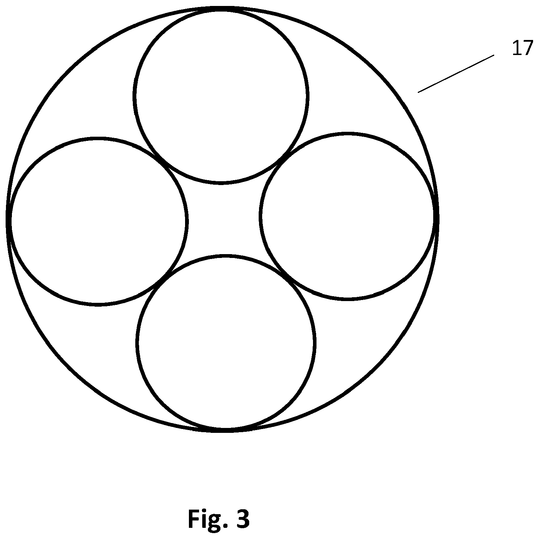

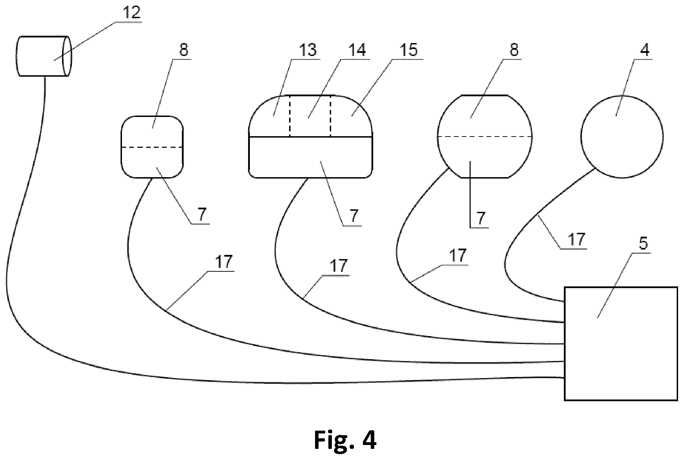

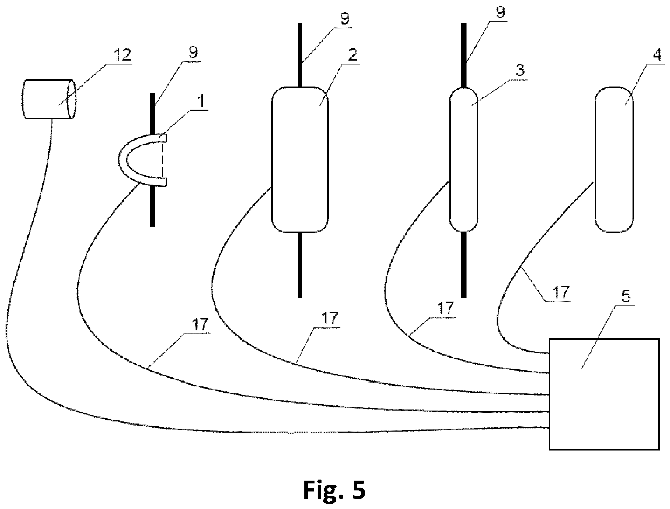

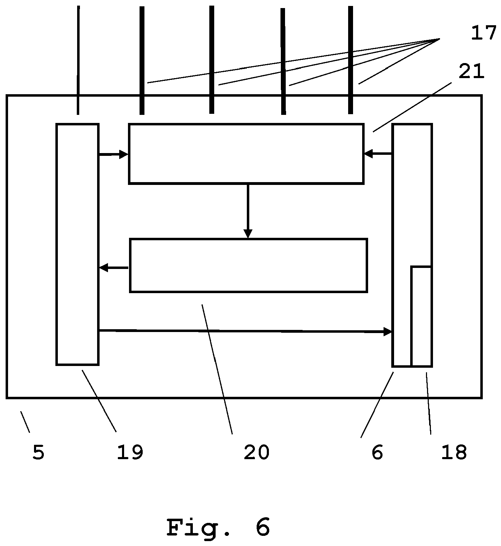

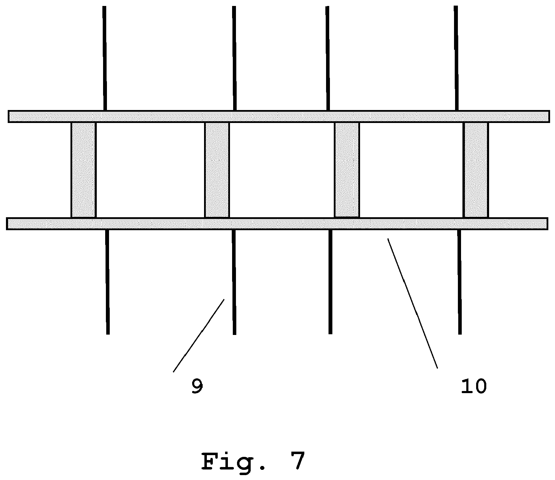

The invention also relates to a mattress system for patient prone positioning, which in the first embodiment consists of inflatable mattresses 1, 2, 3, 4, i.e. a main mattress 1, a chest mattress 2, a pelvic mattress 3, a shin mattress 4, which are connected via separate pipes 17 of a cross-section as shown in Fig. 3, corresponding to the number of cells 7, 8 - including cells 13, 14, 15, connected to a pressure controller 5 connected to a pressure source 6 in the form of a compressor or gas cylinder. In addition, the mattresses 1, 2, 3, have a separate bed-adjoining cell 7 and a separate body-adjacent cell 8, and each of the cells 7, 8 is separately connected to the pressure controller 5. The body-adjacent cell 8 of the chest mattress 2 consists of a pre-jugular cell 13, a chest cell 14, to which the pressure pulse generator 18 is connected and the abdominal cell 15. This is shown in the side view in Fig. 4 and in a top-view in Fig. 5, respectively. As for the pressure controller 5, according to Fig. 6, it consists, for example, of a controller 19 (including a microprocessor and memory) connected to a pressure sensor group 20, as well as a valve group 21 and a pressure source 6, and the pressure source 6 is connected to the pressure sensor unit 20. A preferred variant of the pressure controller 5 is that it additionally carries out the function of a pressure pulse generator 18. On the other hand, the shape in the embodiment of mattresses 1, 2, 3, 4 is as follows: the main mattress 1 has a horseshoe shape, the arms of which are connected by a spacer strip 11 ended with Velcro; the pelvic mattress 3 and the shin mattress 4 have the shape of a cylinder, which is generally an elliptical cylinder. Regardless of the shape, however, in the embodiment, each of the mattresses 1, 2, 3 is provided with fastening straps 9 with Velcro ends, and the shin mattress 4 is optionally equipped (Fig. 5 shows the option without fastening straps 9 for the shin mattress 4). ) with the securing straps 9. An optional element of the system according to the invention is the positioning ladder 10 shown in Fig. 7 for fixing the mattresses 1, 2, 3, 4 thereto and for positioning the mattresses 1, 2, 3, 4 with respect to each other. Of course, these positions are determined by the distance of the respective parts of the patient's body. Such a positioning ladder 10 is fastened to the patient's body by its fastening straps 9 ended with Velcro. The rungs of the positioning ladder 10 are made of plates (e.g. aluminum) attached to the surface of the stringers so that after turning the patient to the prone position, the rung surface is tangent to the bed surface, thanks to which the rungs do not collide with the bed-adjacent cells 7 of the mattresses 1, 2, 3 , 4.

The mattress system for patient prone positioning in the second embodiment is constructed as in the first embodiment, but in addition, to the pressure controller 5 there is connected a pressure measuring system in artificial airways 12, which in particular is a peak pressure sensor of the airways.

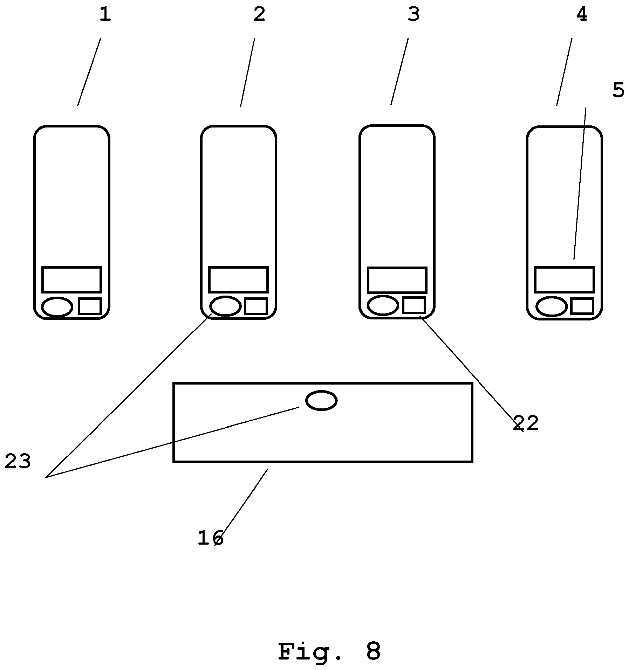

In the third embodiment, a compact version of the mattress system 1, 2, 3, 4 is shown, which differs from the previous examples in that each of the mattresses 1, 2, 3, 4 - as shown in Fig.8, is connected and integrated with a separate pressure controller 5 which is connected and integrated with a separate pressure source 6, each pressure controller 5 having its own autonomous source of electric energy 22, and moreover, each pressure controller 5 has a transceiver module 23 through which it is wirelessly (radio) connected to the central unit 16 also equipped with a transceiver module 23. The central unit 16 is a computer, and in a particular embodiment of the example it is a smartphone. The wireless connection is implemented in the present example in bluetooth or WI-FI technology. Moreover, considering that the mattresses 1, 2, 3, 4 may need to be inflated extremely quickly, and the electric energy source 22, despite sufficiently accumulated energy, may not have sufficient power or the rated power of the compressor may be insufficient in one in the variations of this example, the pressure source 6 is a compressor and additionally a cartridge containing pressurized carbon dioxide.

## Claims

1. A method of patient prone positioning, which consists in placing next to the patient's body - anteriorally, a main mattress by the head, a chest mattress by the chest, a pelvic mattress by the pelvis, a shin mattress by the shins, **characterized by** the fact that at least some of the mattresses used (1, 2, 3, 4) have a separate bed-adjacent cell (7) and a separate body-adjacent cell (8); then the body-adjoining cells (8) are inflated to the state of matching their shape to the patient's body and the patient is placed in the initial prone position, and then the bed-adjoining cells (7) are inflated, evenly lifting individual parts of the body until the influence of the abdominal compression pressure is eliminated.

2. The method according to claim 1, **characterized in that** before prone positioning of the patient and during inflating of the bed-adjoining cells (7), the values of the intra-abdominal pressure are measured with the system measuring the pressure in artificial airways (12), and the bed-adjoining cells ( 7) are inflated to an acceptably small- due to medical conditions, difference between these intra-abdominal pressure values.

3. The method according to claim 1 or 2, **characterized in that** the chest mattress (2) having a body-adjoining cell (8) which has a chest cell (14), there is applied to the chest cell (14) a pulse alternating pressure which is preferably applied every minute for a time10 -15 seconds.

4. The method according to claims 1 to 3, **characterized in that** after placing the mattresses (1, 2, 3, 4) against the patient's body, they are fastened by enveloping the patient's body parts with fastening straps (9) or attached to a positioning ladder (10). ), which is then attached to the patient's body.

5. The method according to claims 1 to 4, **characterized in that** the inflating of the bed-adjoining cells (7) and / or the body-adjoining cells (8) in mattress systems (1, 2, 3, 4) in which a pressure controller (5) is connected to a central unit (16), which is a computer, is carried out until the pressure value is equal to that read from the database, in which the pressure values of the cells (7, 8) for people previously placed in the prone position using mattresses ( 1, 2, 3, 4); the read pressure values apply to people with the most similar values of weight and height within the same sex in relation to the set values of weight and height of the patient currently placed in the prone position.

6. A mattress system for patient prone positioning consisting of inflatable mattresses: main, chest, pelvic, shin, **characterized by** the following: the main mattress (1), the chest mattress (2), the pelvic mattress (3), the shin mattress (4) where the pelvic and shin mattresses are connected to a pressure controller (5) connected to a pressure source (6), at least some of the mattresses (1, 2, 3, 4) have a separate bed-adjoining cell (7) and a separate body-adjoining cell (8), and each of the cells (7, 8) is separately connected to the pressure controller (5).

7. The mattress system according to claim 6, **characterized in that** the mattresses (1, 2, 3) have the separate bed-adjoining cell (7) and the separate body-adjoining cell (8), the body-adjoining cell (8) of the chest mattress (2) consisting of: a pre-jugular cell (13), a chest cell (14), and a pre-abdominal cell (15).

8. The mattress system according to claim 6 or 7, **characterized in that** the pressure controller (5) comprises a controller (19) connected to a pressure sensor assembly (20), and to a valve assembly (21) and the pressure source (6), and the pressure source (6) is connected to the pressure sensor unit (20).

9. The mattress system according to claim 6 to 8, **characterized in that** a pressure pulse generator (18) is connected to the chest cell (14).

10. The mattress system according to claims 6 to 9, **characterized in that** the pressure pulse generator (18) is contained in the pressure controller (5) to which an artificial airway pressure measuring system (12) is preferably connected.

11. The mattress system according to claims 6 to 10, **characterized in that** the artificial airway pressure measuring system (12) is a peak airway pressure sensor.

12. The mattress system according to claims 6 to 11, **characterized in that** the main mattress (1) is shaped like a horseshoe, the arms of which are connected by a spacer strip (11), and the pelvic mattress (3) and the shin mattress (4) have the shape of a cylinder - including an elliptical cylinder

13. The mattress system according to claims 6 to 12, **characterized in that** the mattresses (1, 2, 3, 4) have fastening bands (9) which preferably have Velcro.

14. The mattress system according to claims 6 to 13, **characterized in that** the mattresses (1, 2, 3, 4) are connected to the pressure controller (5) through the conduits (17) distributed according to the number of connected cells (7, 8) - including the cells (13, 14, 15).

15. The mattress system according to claims 6 to 14, **characterized in that** each of the mattresses (1, 2, 3, 4) is connected and integrated with the separate pressure controller (5) which is connected and integrated with the separate pressure source (6) wherein each pressure controller (5) having its own autonomous source of electric energy (22), and moreover, each pressure controller (5) has a transceiver module (23) through which it is wirelessly connected to a central unit (16).
